# EUROPEAN PATENT SPECIFICATION

(11) **EP 0 518 050 B1**
(45) Date of publication and mention of the grant of the patent: **10.07.1996**
(21) Application number: 92107771.5
(22) Date of filing: 08.05.1992
(51) Int. Cl.: A61M 1/10, F04D 29/22

(54) **Liquid pump apparatus**
Flüssigkeitspumpe
Pompe à liquide

(30) Priority: 10.05.1991 JP 135943/91; 11.09.1991 JP 232031/91
(43) Date of publication of application: 16.12.1992
(73) Proprietor: TERUMO KABUSHIKI KAISHA, Tokyo (JP)
(72) Inventor: Kijima, Toshihiko, c/o Terumo Kabushiki Kaisha, Ashigarakami-gun, Kanagawa-ken (JP); Horiuchi, Kunio, c/o Terumo Kabushiki Kaisha, Ashigarakami-gun, Kanagawa-ken (JP); Oshiyama, Hiroyuki, c/o Terumo Kabushiki Kaisha, Ashigarakami-gun, Kanagawa-ken (JP)
(74) Representative: Casalonga, Axel

(56) References cited:
- BE-A- 525 787
- US-A- 1 406 297
- US-A- 3 107 625
- US-A- 4 135 253
- US-A- 4 984 972

## Description

The present invention relates to mainly a liquid pump apparatus for transporting a physiological fluid such as blood and, more particularly, to a liquid pump apparatus capable of suppressing turbulence of a liquid to be transported and enhancing good pump characteristics at a low speed.

Known turbo pump apparatuses for transporting a physiological fluid such as blood or plasma are described in US-A-4,589,822 and US-A-3,864,055. These pumps are turbo pumps for feeding blood using centrifugal forces. The former turbo pump generates the centrifugal force upon rotation of a general open type multi-blade vane assembly, while the latter turbo pump generates the centrifugal force by utilizing a friction force between a plurality of conical rotators.

The most important things for blood transportation are to prevent solid components such as erythrocytes and platelets in the blood from being destroyed and to prevent the blood from coagulating as a result of contamination of foreign objects or the like. The most important cause for destruction of the above solid components is blood turbulence during transportation. The blood pump disclosed in US-A-3,864,055 is a pump for applying the centrifugal force to the fluid by the friction force between the plurality of conical rotators. This pump is known as a pump for minimizing turbulence in a blood path between the rotators and hence destruction of solid components (Hydrodynamical and Hemodynamical Evaluation of Rotary Blood Pumps, Inter. Workshop on Rotary Blood Pumps: Vienna, 1988, pp. 76-81).

This blood pump, however, has pump efficiency lower than that of a general pump using vanes. In order to obtain a given pump head, the above blood pump requires a higher speed than that of a vane pump having the same size. For this reason, local heating occurs in a seal portion of a rotating shaft, and the blood around the rotating shaft is denatured and coagulated. In order to obtain the same flow rate as the vane pump having the same size, the blood pump requires a plurality of rotators, and the amount of blood filled in the pump is undesirably increased.

On the other hand, the blood pump described in US-A-4,589,822 uses an open type multi-blade vane assembly having a large opening at its center to reduce local heating at the seal portion of the shaft, so that the blood speed near the seal portion is increased. In addition, a heat sink structure is added to this pump. In the open type vane pump of this type, however, the blood flow tends to be separated from the vanes, and a counter flow tends to occur between the vanes. So, the blood flow to be turbulent, and solid components (e.g., erythrocytes) in the blood tend to be destroyed.

US-A-1 406 297, discloses a liquid pump having closed liquid paths within the rotator and being provided for allowing variation or adjustment of the capacity or output of the pump. It comprises a rather high casing or chamber 1 and an impeller or runner 2 rotatively mounted in the upper part of the casing. Liquid introduced therein is forced therethrough into the chamber 1 by centrifugal action. Operation of this pump is preferred in such a manner that a body of air or other elastic medium is retained in the chamber 1 so that the impeller rotates in contact with such a body of air thereby minimizing the frictional loss. When the chamber is filled with the liquid, the air is correspondingly compressed. The impeller comprises two disc shaped portions 15 and 16, between which liquid passages are formed by spacing means 17. By adjusting the position of the spacing means 17 the size or cross-sectional area of the outlet end portions of the passages 14 can be varied. The spacing members 17 are so shaped that the channels 14 are directed radially outward and rearward and the channels 14 taper or decrease in cross-section outwardly from the inlet means. Herein the cross-sectional area of the liquid path is rapidly diminished toward the outlet end. In any case, the liquid is directed such that it falls substantially "dead" or without any considerable velocity relative to the walls of the casing 1. This centrifugal pump cannot be applied for transporting physiological fluids such as blood, since the presence of bubbles or air is readily taken into account. However, air should be avoided or eliminated from blood for preventing coagulation thereof. For a similar reason, the presence of frictional forces has to be avoided, since otherwise solid components of blood can be destroyed.

It is an object of the present invention to provide a liquid pump apparatus capable of suppressing turbulence of a body fluid to be transported without increasing the amount of the fluid filled in the pump and capable of obtaining good pumping characteristics at low rotation speed.

According to an aspect of the present invention, a liquid pump apparatus comprises a housing forming a liquid chamber, an inlet formed at an upper central portion of the housing and communicating with a liquid source and the liquid chamber and an outlet formed in a peripheral portion of the housing and communicating with the liquid chamber. A substantially cylindrical rotator is arranged in the liquid chamber and has an upper cover and a shroud. A plurality of liquid paths are radially formed between the upper cover and the shroud to substantially uniformly distribute liquid from a central portion of the rotator to a peripheral portion thereof. Bearing means are rotatably supporting the rotator with respect to the housing, and means are provided for applying a rotational force to the rotator. Each of the plurality of liquid paths is formed so that a cross-sectional area thereof is substantially uniform or reduced in the direction of liquid flow. The liquid chamber is substantially cylindrical, forming an annular space between the housing and the rotator, the inlet being provided in the axial direction of the liquid chamber. A projection having an inclined surface is formed at an upper central portion of the shroud and is located immediately below the inlet. The upper cover and the upper housing wall are formed in such a manner that liquid present in the liquid paths is guided to the outlet in the upper portion of the housing.

The shape of the liquid path is preferably arcuate or straight.

The central axis of the liquid path preferably crosses that of the rotator at an angle falling within the range of about 75° to 90°.

This invention can be more fully understood from the following detailed description when taken in conjunction with the accompanying drawings, in which:
Fig. 1 is a longitudinal sectional view of a blood pump according to the first embodiment of the present invention;
Fig. 2 is a cross-sectional view of the blood pump of the first embodiment when taken along a rotator;
Fig. 3 is a graph showing hemolytic characteristics of the blood pump of the first embodiment and a conventional blood pump (control);
Fig. 4 is a graph showing pumping characteristics of the blood pump of the first embodiment;
Fig. 5 is a longitudinal sectional view of a blood pump according to the second embodiment of the present invention;
Fig. 6 is a cross-sectional view of the second pump of the second embodiment when taken along a rotator;
Fig. 7 is a graph showing pumping characteristics of the blood pump of the second embodiment;
Fig. 8 is a graph showing pumping characteristics of the blood pump of the third embodiment;
Fig. 9 is a longitudinal sectional view of a blood pump according to the third embodiment of the present invention;
Fig. 10 is a cross-sectional view of the blood pump of the third embodiment when taken along a rotator; and
Fig. 11 is a graph showing pumping characteristics of the blood pump of the third embodiment.

Various embodiments using transfusion blood pumps to which the present invention is applied will be described below.

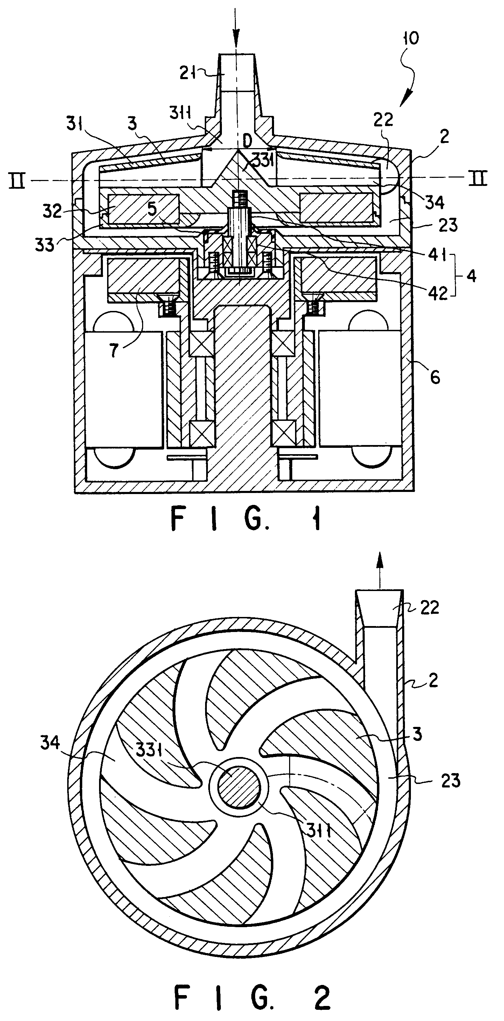

As shown in Figs. 1 and 2, a portion of a blood pump 10 of the first embodiment which guides blood is covered with a housing 2. A blood inlet 21 is formed at the upper central portion of the housing 2 and communicates with a blood reservoir (not shown). An almost cylindrical chamber 23 is formed in the housing 2. A rotator 3 is provided in the chamber 23.

A blood outlet 22 is formed in a peripheral portion of the housing 2, and its flow path is directed tangentially to the rotator 3.

Blood is supplied to the housing 2 from the blood inlet 21. A centrifugal force is applied to the blood inside the chamber, and the blood is delivered from the blood outlet 22.

The rotator 3 comprises a cover 31 and a shroud 33 in which a multipole disk-like driven magnet 32 is embedded. A plurality (six in this embodiment) of liquid paths 34 are radially formed between the cover 31 and the shroud 33. A blood flow inlet 311 is formed in the cover 31 to guide and flow, to the liquid paths 34, the blood supplied from the blood inlet 21 to the housing 2.

More specifically, the blood supplied to the housing 2 is distributed to the liquid paths 34 through the blood flow inlet 311, receives a centrifugal force upon rotation of the rotator 3, and is delivered outside the housing through the blood outlet 22.

Each distribution path 34 has a tubular structure having a rectangular cross-sectional shape and an arcuated shape in its axial direction. The cross-sectional shape of the liquid path 34 is not limited to the illustrated one, but may be circular or a polygonal shape other than a rectangular shape.

Each distribution path 34 is formed so that its cross-sectional area is monotonously decreased along the blood flow within the path. For this reason, the blood flow in the path is accelerated, and the flow tends not to be separated from the walls of paths, thereby suppressing turbulence. With the above arrangement, a sufficiently high pumping ability (pump head) can be obtained at low speed of the pump, without increasing of the blood volume filled in the pump, so that local heating of the blood which is caused by friction of a seal unit 5 (to be described later) can be appropriately suppressed.

Each liquid path 34 is preferably formed so that its central axis defines an angle falling within the range of about 75° to 90° (90° in the structure in Fig. 1) with respect to the central axis of the rotator 3. In this arrangement, while the blood is filled in the pump, bubbles present in the liquid paths 34 are guided to the upper portion of the housing 2 and are easily removed from the blood inlet 21.

The rate of reduction of the cross-sectional area of each liquid path 34 (i.e., the ratio of the cross-sectional area of the inlet end of the path to that of the outlet end of the path) is preferably set to be about 50% or less.

The liquid paths 34 are preferably formed at substantially equal angular intervals. The number of liquid paths 34 is not limited to any specific value but can preferably fall within the range of 2 to 12.

A conical projection 331 is formed on the shroud 33 at a position opposite to the blood flow inlet 311 to divide the blood supplied from the blood flow inlet 311 so as to guide the blood to the respective liquid paths 34. The inclined surface of the projection 331 preferably has an angle falling within the range of about 10° to 80° to the central axis. The bottom portion of the projection 331 preferably has a diameter falling within the range of about 1/1 to 1/4 the diameter D of the blood flow inlet 311.

A bearing 4 will be described below.

The bearing assembly 4 rotatably supports the rotator 3 in the housing. The bearing assembly 4 comprises a shaft 41 fixed on the shroud 33 and a ball bearing 42 mounted on the inner bottom of the housing 2. The ball bearing 42 and the housing 2 are liquid-tightly separated from each other by the seal member 5.

The seal member 5 is not limited to a lip seal illustrated in Fig. 1, but may be a face seal using an elastic member and a counter face, a mechanical seal using a slidable member made of low friction material and a counter face, or the like.

A sliding bearing or the like may be used in place of the ball bearing 42.

A rotational torque for driving the rotator 3 is transmitted to the driven magnet 32 from a driving magnet 7 coaxially fixed on the rotating shaft of an external motor 6. The rotator 3 is rotated upon rotation of the external motor 6, and the blood supplied from the blood inlet 21 passes through the blood flow inlet 311 of the rotator 3 and then passes through the plurality of liquid paths 34 arranged radially thus receiving the centrifugal force from these paths 34. The blood is delivered from the blood outlet 22. It is possible to arrange the external motor 6 by a flat brushless motor structure in which only a flat stator coil is used and the driven magnet 32 is directly driven by the stator coil.

A detailed arrangement of the blood pump 10 of the first embodiment will be described below.

### [First Embodiment]

### 〈Housing〉

| | |
|---|---|
| Material | Acrylic resin |
| Inner Diameter of Housing | 84 mm |
| Amount of Filled Blood | 50 cc |
| Inner Diameter of Blood Inlet | 8 mm |
| Inner Diameter of Blood Outlet | 8 mm |

### 〈Rotator〉

| | |
|---|---|
| Material | Polycarbonate resin |
| Outer Diameter | 74 mm |
| Inner Diameter of Blood Flow Inlet | 19 mm |
| Number of liquid Paths (radially arranged at equal angular intervals) | 6 |
| Cross-sectional Area of Inlet Opening of Liquid Path | 50 mm² |
| Cross-sectional Area of Outlet Opening of Liquid Path | 33 mm² |
| Angle between Central Axis of Liquid path and That of Rotator Body | 90° |
| Angle between Inclined Surface of Projection and Central Axis | 45° |
| Diameter of Bottom Portion of Projection | 15 mm |
| Driven Magnet | 6-pole magnetized ferrite magnet (outer diameter: 70 mm; inner diameter: 32 mm; and thickness: 8 mm) |

### 〈External Drive Unit〉

| | |
|---|---|
| Motor | 90 W brushless DC motor |
| Driving Magnet | 6-pole magnetized ferrite magnet (outer diameter: 70 mm; inner diameter: 32 mm; thickness: 10 mm; and Distance from Driven magnet 8.5 mm) |

### [Control]

A blood pump model BP80 (available from Biomedicus Corp.) described in U.S.P. No. 3,864,055 was used. The amount of blood filled in this blood pump was 80 cc.

### [Measurement of Pump Speed]

These pump apparatuses were operated using 1.8 ℓ of blood having a hematocrit rate of 43% as a liquid to be transported at a pump head of 400 mmHg and a flow rate of 3 ℓ/min. The speed of the blood pump of the first embodiment at this operating point was 2,370 rpm, while the speed of the blood pump of the comparative example at above operating point was 2,900 rpm. The amount of blood filled in the blood pump of the present invention was greatly reduced as that of the comparative example. Therefore, the blood pump of the first embodiment was confirmed to be operable at low speed (lower than that of the conventional pump) to obtain the same pressure head.

### [Measurement of Hemolytic Rate]

These pump apparatuses were operated using 1.8 ℓ of blood having a hematocrit rate of 43% as a liquid to be transported at a pump head of 400 mmHg and a flow rate of 3 ℓ/min, and hemolytic rates (i.e., free hemoglobin concentration in the blood) over time were measured.

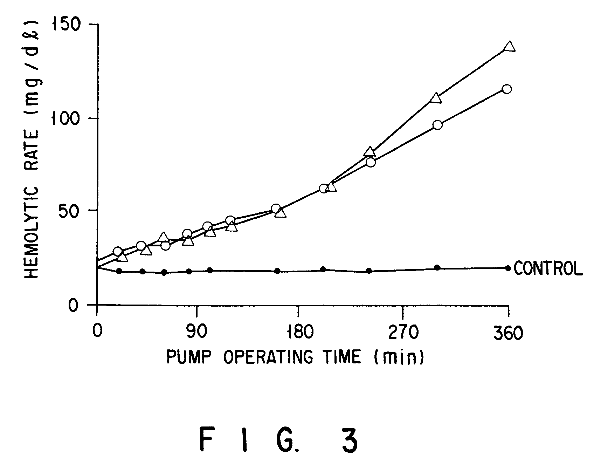

Fig. 3 is a graph showing measurement results of the first embodiment and the conventional pump in which the pump operating time is plotted along the abscissa and the hemolytic rates are plotted along the ordinate.

The control represented by dots in Fig. 3 represents a hemolytic rate of the blood which has not yet been transported. Blank circles represent the result using the pump of the first embodiment, and blank triangles represent the result using the conventional pump.

As is apparent from Fig. 3, the blood pump of the first embodiment of the present invention was confirmed to have almost the same low hemolytic rate as that of the conventional pump.

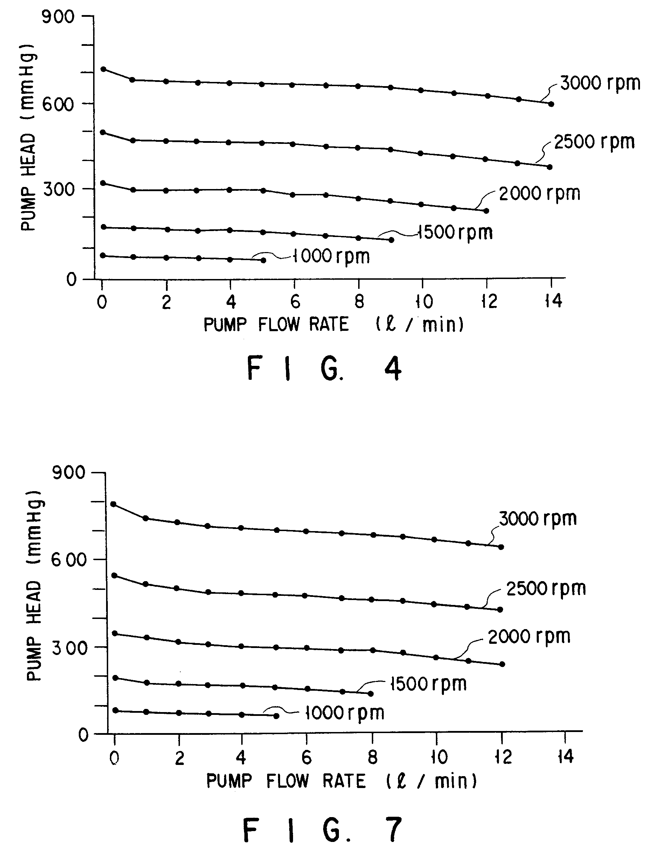

Fig. 4 is a graph showing the test results of pumping characteristics of the first embodiment when the pump speed is changed in the range of 1,000 to 3,000 rpm. The pump flow rates are plotted along the abscissa in Fig. 4, and the pump heads are plotted along the ordinate.

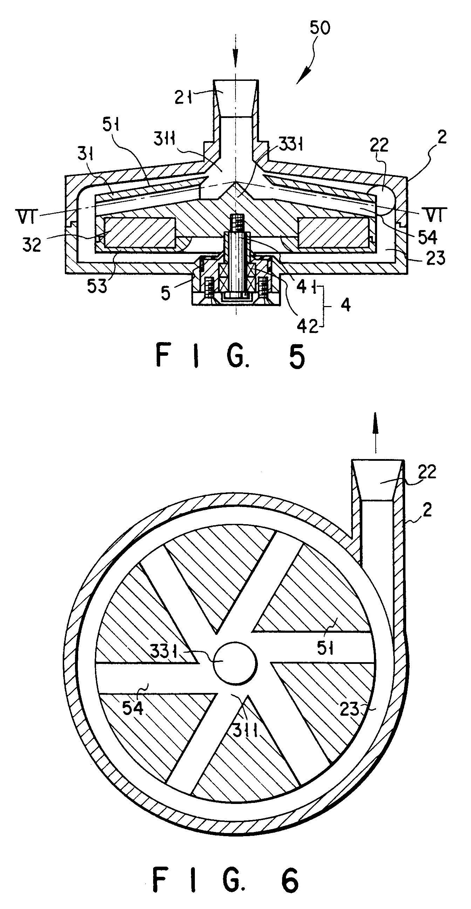

A blood pump according to the second embodiment will be described with reference to Figs. 5 and 6.

The same reference numerals as in the first embodiment denote the same parts in the second embodiment, and a detailed description thereof will be omitted.

A pump 50 of the second embodiment is substantially the same as the pump 10 of the first embodiment, except that blood paths 54 are straight and have constant cross-sectional areas along the blood flow in the paths. With the above arrangement, as compared with the pump 10 in the first embodiment, the amounts of blood filled in the distribution paths can be almost equal to each other, and better pump characteristics can be obtained. In addition, each blood path 54 is formed such that an angle i between the central axis of a rotator 51 and that of the paths 54 is set to be 80°.

A detailed arrangement of the blood pump 50 of the second embodiment will be described below.

### [Second Embodiment]

### 〈Housing〉

| | |
|---|---|
| Material | Acrylic resin |
| Inner Diameter of Housing | 84 mm |
| Amount of Filled Blood | 47 cc |
| Inner Diameter of Blood Inlet | 8 mm |
| Inner Diameter of Blood Outlet | 8 mm |

### 〈Rotator〉

| | |
|---|---|
| Material | Polycarbonate resin |
| Outer Diameter | 74 mm |
| Inner Diameter of Blood Flow Inlet | 19 mm |
| Number of Liquid Paths (radially arranged at equal angular intervals) | 6 |
| Cross-sectional Area of Inlet Opening of Liquid Path | 32 mm² (IH 4 mm × IW 8 mm) |
| Cross-sectional Area of Outlet Opening of Liquid Path | 32 mm² (OH 4 mm × OW 8 mm) |
| Angle between Central Axis of Liquid Path and That of Rotator | 80° |
| Angle between Inclined Surface of Projection and Central Axis | 45° |
| Diameter of Bottom Portion of Projection | 10 mm |
| Driven Magnet | 6-pole magnetized ferrite magnet (outer diameter: 70 mm inner diameter: 32 mm; and thickness: 8 mm) |

### 〈External Drive Unit〉

The same as in the first embodiment.

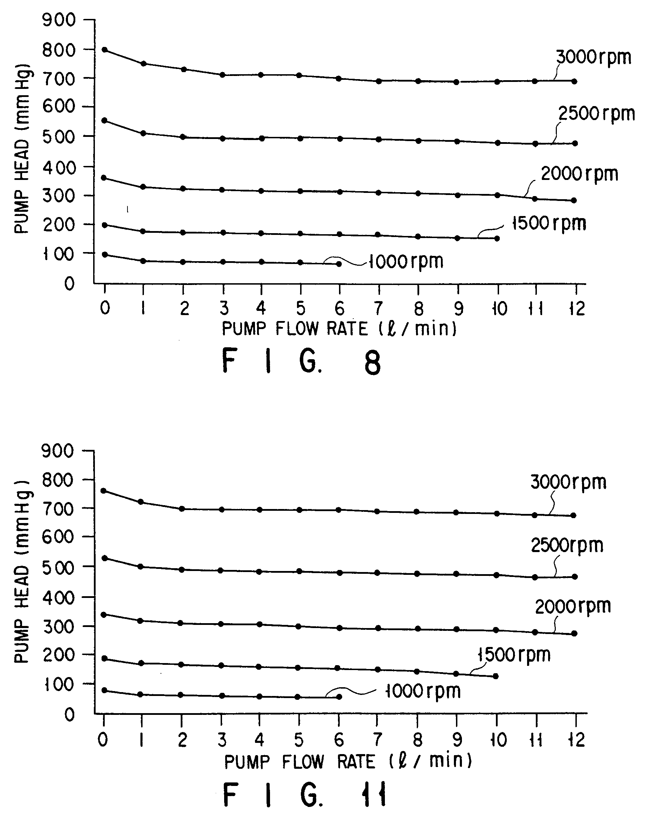

Fig. 7 is a graph showing the test results of pumping characteristics of the second embodiment when the pump speed is changed in the range of 1,000 to 3,000 rpm. The pump flow rates are plotted along the abscissa in Fig. 7, and the pump heads are plotted along the ordinate. The pump characteristics were measured using a glycerin solution, viscosity of 4 c.p.

A physiological saline at a temperature of 25°C was transported using two kinds of the blood pumps 70 of the third embodiment, and their pumping characteristics were measured. The measurement was performed by changing the pump speeds and the pump flow rates. The results are shown in Fig. 8 and Fig. 11. Liquid paths of third embodiment of which results shown in Fig. 8 are straight as same as the second embodiment. Liquid paths of third embodiment of which results shown in Fig. 11 are spread at a gradient of about 1° along the blood flow. As is apparent from Figs. 8 and 11, both of two kinds of blood pumps 70 exhibited good pump characteristics.

Fig. 8 is a graph showing the test results of pumping characteristics of the third embodiment when the pump speed is changed in the range of 1,000 to 3,000 rpm. The pump flow rates are plotted along the abscissa in Fig. 8, and the pump heads are plotted along the ordinate. The pumping characteristics were measured using a physiological saline at a temperature of 25°C.

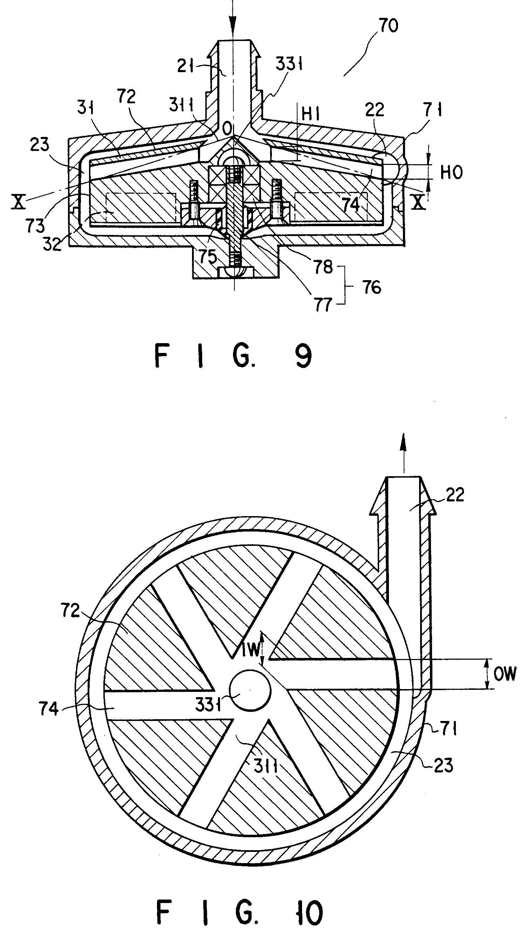

A blood pump according to the third embodiment of the present invention will be described with reference to Figs. 9 and 10. The same reference numerals as in the first and second embodiments denote the same parts in the third embodiment, and a detailed description thereof will be omitted.

The pump 70 of the third embodiment is substantially the same as the pump 50 of the second embodiment. A cover 31 and a shroud 73 will be formed integrally with each other to constitute a rotator 72, if an inclined portion will be formed as an escape taper for molding.

In addition, since the rotator 72 of the pump 70 of the third embodiment is made of an integral body, the blood path is seamless to rarely cause thrombus.

A detailed arrangement of the blood pump 70 of the third embodiment will be described below.

In a seal mechanism 76 of the third embodiment, a bearing 78 is mounted at an upper end portion of a shaft 77.

In a housing 71 of the third embodiment, the proximal portion of the blood outlet 22 slightly extends outward.

### [Third Embodiment]

### 〈Housing〉

| | |
|---|---|
| Material | Acrylic resin |
| Inner Diameter of Housing | 84 mm |
| Amount of Filled Blood | 47 cc |
| Inner Diameter of Blood Inlet | 8 mm |
| Inner Diameter of Blood Outlet | 8 mm |

### 〈Rotator〉

| | |
|---|---|
| Material | Polycarbonate resin |
| Outer Diameter | 78 mm |
| Inner Diameter of Blood Flow Inlet | 19 mm |
| Number of Liquid Paths (radially arranged at equal angular intervals) | 6 |
| Cross-sectional Area of Inlet Opening of Liquid Path | 28.1 mm² (IH 3.7 mm × IW 7.6 mm) |
| Cross-sectional Area of Outlet Opening of Liquid Path | 38.7 mm² (OH 4.5 mm × OW 8.6 mm) |
| Angle between Central Axis of Distribution Path and That of Rotator Body | 80° |
| Angle between Inclined Surface of Projection and Central Axis | 45° |
| Diameter of Bottom Portion of Projection | 10 mm |
| Driven Magnet | 6-pole magnetized ferrite magnet (outer diameter: 70 mm; inner diameter: 32 mm; and thickness: 8 mm) |

### 〈External Drive Unit〉

The same as the first embodiment.

### [Measurement of Pump Characteristics]

A glycerin solution of which viscosity was 4 c.p., was transported using the blood pumps 10 and 50 of the first and second embodiments, and their pump characteristics were measured. The measurement was performed by changing the pump speeds and pump flow rates. The results are shown in Fig. 3 (first embodiment) and Fig. 7 (second embodiment). As is apparent from Figs. 3 and 7, both the blood pumps 10 and 50 exhibited good pumping characteristics.

The present invention is not limited to the pump 10 arranged such that the blood paths 34 are arcuated and their cross-sectional areas are monotonously decreased, as shown in Fig. 1, and the pump 50 arranged such that the liquid paths 54 are straight and their cross-sectional areas are almost constant along the blood flows in the paths 54, as shown in Fig. 5. The same effects as described above can be obtained in a pump arranged such that the liquid paths are straight and their cross-sectional areas are monotonously decreased, or in a pump arranged such that the liquid paths are arcuated and their cross-sectional areas are kept almost constant.

The bearing structures are not limited to the ones shown in Figs. 1 and 5. For example, the shaft 41 may be fixed in the housing 2, or the bearing 42 and the seal unit 5 may be arranged in the shroud 33 (as shown in Fig. 9). In addition, the driving unit and the driven unit may be directly connected to each other without going through magnets.

A liquid to be transported is not limited to blood. As long as a liquid contains a material which tends to be denatured by heat, the present invention is applicable to any such liquid.

Since a liquid pump according to the present invention is arranged as described above, the liquid supplied from the liquid inlet passes through a liquid flow portion of a rotator and then passes through a plurality of radial liquid paths, thereby receiving a centrifugal force. The liquid is then delivered from the liquid outlet. At this time, since the liquid paths are formed such that their cross-sectional areas are kept almost constant or monotonously decreased along the fluid flows in the paths, the resultant liquid flows become substantially constant-speed flows or accelerated flows, and the flows tend not to be separated from the walls of the liquid paths, thereby preventing turbulence without increasing the amount of the liquid filled in the pump. Therefore, damage to the solid components in blood can be prevented. In addition, if the liquid paths are straight, the amount of a body fluid filled in the pump can be further reduced without degrading the pump characteristics. Since the central axis of each liquid path forms an angle falling within the range of about 75° to 90° with respect to that of the rotator, bubbles can be effectively removed without degrading the pumping characteristics.

As has been described above, a liquid pump apparatus according to the present invention has a liquid inlet and a liquid outlet. The pump apparatus comprises a housing having an almost cylindrical chamber therein, a plurality of radial liquid paths, a rotator rotatably mounted in the housing, and a bearing for rotatably supporting the rotator. In this pump apparatus, the liquid paths are formed such that their cross-sectional areas are almost constant or reduced along the liquid flows in the paths. The liquid supplied from the liquid inlet passes through the liquid flow portion of the rotator and then passes through the plurality of radial liquid paths, thereby receiving a centrifugal force. The liquid is then delivered from the liquid outlet. At this time, since the liquid paths are formed such that their cross-sectional areas are almost constant or monotonously reduced along the liquid flows in the paths, the resultant liquid flows become substantially constant-speed liquid flows or accelerated liquid flows. As a result, the flow is rarely separated from the walls of the liquid paths, the turbulence can be prevented without increasing the amount of the liquid filled in the pump, and the damage to the solid components in the blood can be prevented. In addition, if the liquid paths are straight, the amount of the liquid filled in the pump can be further reduced without degrading the pumping characteristics. Since the central axis of each liquid path forms an angle falling within the range of about 75° to 90° with respect to that of the rotator, bubbles can be effectively removed without degrading the pumping characteristics.

## Claims

1. A liquid pump apparatus comprising:
- a housing (2, 71) forming a liquid chamber (23);
- an inlet (21) formed at an upper central portion of the housing and communicating with a liquid source and the liquid chamber (23);
- an outlet (22) formed in a peripheral portion of the housing and communicating with the liquid chamber (23);
- a substantially cylindrical rotator (3, 51, 72) arranged in the liquid chamber (23) and having an upper cover (31) and a shroud (33, 53, 73);
- a plurality of liquid paths (34, 54, 74) radially formed between the upper cover (31) and the shroud (33, 53, 73) to uniformly distribute liquid from a central portion of the rotator (3, 51, 72) to a peripheral portion thereof;
- bearing means (42, 78) rotatably supporting the rotator (3, 51, 72) with respect to the housing (2, 71); and
- means (6) for applying a rotational force to the rotator (3, 51, 72),
- wherein the plurality of liquid paths (34, 54, 74) is formed so that a cross-sectional area thereof is substantially uniform or reduced in the direction of liquid flow,
characterised in that
- the liquid chamber (23) is substantially cylindrical, forming an annular space between the housing (2) and the rotator (3, 51, 72),
- the inlet (21) is provided in the axial direction of the liquid chamber (23),
- a projection (331) having an inclined surface is formed at an upper central portion of the shroud (33, 53, 73) and is located immediately below the inlet (21),
- the upper cover (31) and the upper housing wall are formed in such a manner that liquid present in the liquid paths (34) is guided to the outlet in the upper portion of the housing (2).

2. The apparatus according to claim 1, characterised in that each of the liquid paths (34) has an arcuate shape.

3. The apparatus according to claim 1 or 2, characterised in that each of the liquid paths (54, 74) has a straight shape.

4. The apparatus according to any of claims 1 to 3, characterised in that each of the liquid paths (34, 54, 74) is formed such that a central axis thereof is inclined at an angle of 75° to 90° with respect to a central axis of the rotator (3, 51, 72).

5. The apparatus according to any of claims 1 to 4, characterised in that each of the plurality of liquid paths has a cross-sectional area gradually diminished toward the outlet end such that the cross-sectional area at the outlet end is at most 50 % of that at the inlet end.

## Patentansprüche

1. Flüssigkeitspumpvorrichtung, umfassend:
- ein Gehäuse (2,71), das eine Flüssigkeitskammer (23) bildet;
- einen Einlaß (21), der an einem oberen zentralen Abschnitt des Gehäuses gebildet ist und mit einer Flüssigkeitsquelle und der Flüssigkeitskammer (23) in Verbindung steht;
- einen Auslaß (22), der in einem Umfangsabschnitt des Gehäuses gebildet ist und mit der Flüssigkeitskammer (23) in Verbindung steht;
- einen im wesentlichen zylindrischen Rotator (3, 51, 72), der in der Flüssigkeitskammer (23) angeordnet ist und eine obere Abdeckung (31) und ein Abdeckband (33, 53, 73) aufweist;
- eine Anzahl von Flüssigkeitspfaden (34, 54, 74), die zwischen der oberen Abdeckung (31) und dem Abdeckband (33, 53, 73) radial gebildet sind, um Flüssigkeit von einem zentralen Abschnitt des Rotators (3, 51, 72) zu einem Umfangsabschnitt von diesem gleichmäßig zu verteilen;
- Lagermittel (72, 78) die dem Rotator (3, 51, 72) in bezug auf das Gehäuse (2, 71) drehbar halten; und
- Mittel (6) zum Aufbringen einer Rotationskraft auf den Rotator (3, 51, 72),
- wobei die Anzahl der Flüssigkeitspfade (34, 54, 74) so gebildet ist, daß eine Querschnittsfläche von ihnen im wesentlichen gleichmäßig oder in der Richtung der Flüssigkeitsströmung reduziert ist,
dadurch gekennzeichnet daß,
- die Flüssigkeitskammer (23) im wesentlichen zylindrisch ist, wobei sie einen ringförmigen Raum zwischen dem Gehäuse (2) und dem Rotator (3, 51, 72) bildet,
- der Einlaß (21) in der axialen Richtung der Flüssigkeitskammer (23) vorgesehen ist,
- ein Vorsprung (331) mit einer geneigten Fläche an einem oberen zentralen Abschnitt des Abdeckbandes (33, 53, 73) gebildet ist und sich unmittelbar unterhalb des Einlasses (21) befindet,
- die obere Abdeckung (31) und die obere Gehäusewand auf solche Weise gebildet sind, daß in den Flüssigkeitspfaden (34) vorhandene Flüssigkeit zu dem Auslaß in dem oberen Abschnitt des Gehäuses (2) geführt ist.

2. Vorrichtung nach Anspruch 1, dadurch gekennzeichnet, daß jeder der Flüssigkeitspfade (34) eine gebogene Form aufweist.

3. Vorrichtung nach Anspruch 1 oder 2, dadurch gekennzeichnet, daß jeder der Flüssigkeitspfade (54, 74) eine gerade Form aufweist.

4. Vorrichtung nach einem der Ansprüche 1 bis 3, dadurch gekennzeichnet, daß jeder der Flüssigkeitspfade (34, 54, 74) auf solche Weise gebildet ist, daß eine zentrale Achse von diesem in einem Winkel von 75° bis 90° in bezug auf eine zentrale Achse des Rotators (3, 51, 72) geneigt ist.

5. Vorrichtung nach einem der Ansprüche 1 bis 4, dadurch gekennzeichnet, daß jeder der Anzahl der Flüssigkeitspfade eine Querschnittsfläche aufweist, die allmählich zu dem Auslaßende verjüngt ist derart, daß die Querschnittsfläche am Auslaßende höchstens 50% derjenigen am Einlaßende ist.

## Revendications

1. Appareil formant pompe à liquide, qui comprend :
- une enceinte (2, 71) formant une chambre à liquide (23),
- une entrée (21) formée en la partie supérieure centrale de l'enceinte et communiquant avec une source de liquide et avec la chambre à liquide (23),
- une sortie (22) formée dans une partie périphérique de l'enceinte et communiquant avec la chambre à liquide (23),
- un rotateur (3, 51, 72), sensiblement cylindrique, disposé dans la chambre à liquide (23) et comportant un couvercle supérieur (31) et un capot (33, 53, 73),
- une pluralité de passages pour liquide (34, 54, 74) définis radialement entre le couvercle supérieur (31) et le capot (33, 53, 73), pour répartir de façon sensiblement uniforme le liquide de la partie centrale du rotateur (3, 51, 72) à sa partie périphérique,
- des moyens formant paliers (42, 78) supportant en rotation le rotateur (3, 51, 72) par rapport à l'enceinte (2, 71), et
- des moyens (6) pour appliquer une force de rotation au rotateur (3, 51, 72),
sachant que chacun des passages pour liquide (34, 54, 74) de la pluralité est formé de telle sorte que sa section transversale soit sensiblement uniforme ou réduite dans la direction d'écoulement du liquide,
caractérisé en ce que :
- la chambre à liquide (23) est sensiblement cylindrique et forme un espace annulaire entre l'enceinte (2) et le rotateur (3, 51, 72),
- l'entrée (21) est placée dans la direction axiale de la chambre à liquide (23),
- une saillie (331) comportant une surface inclinée est formée en la partie supérieure centrale du capot (33, 53, 73), en étant située immédiatement en-dessous de l'entrée (21),
- le couvercle supérieur (31) et la paroi supérieure de l'enceinte sont formés de telle façon que le liquide présent dans les passages pour liquide (34) soit guidé vers la sortie se trouvant dans la partie supérieure de l'enceinte (2).

2. Appareil selon la revendication 1, caractérisé en ce que chaque passage pour liquide (34) a une forme courbe.

3. Appareil selon la revendication 1, caractérisé en ce que chaque passage pour liquide (54, 74) a une forme rectiligne.

4. Appareil selon l'une quelconque des revendications 1 à 3, caractérisé en ce que chaque passage pour liquide (34, 54, 74) est formé de telle sorte que son axe central soit incliné par rapport à l'axe central du rotateur (3, 51, 72) d'un angle valant de 75 à 90°.

5. Appareil selon l'une quelconque des revendications 1 à 4, caractérisé en ce que chaque passage pour liquide a une section qui diminue progressivement en direction de l'extrémité de sortie, de sorte que la section à l'extrémité de sortie est au plus égale à 50% de celle à l'extrémité d'entrée.
